# FASCICULE DE BREVET EUROPEEN

(11) **EP 4 114 315 B1**
(45) Date de publication et mention de la délivrance du brevet: **13.12.2023**
(21) Numéro de dépôt: 20845599.8
(22) Date de dépôt: 16.12.2020
(51) Int. Cl.: A61F 2/32, A61F 2/34

(54) **IMPLANT ACÉTABULAIRE POUR PROTHÈSE DE HANCHE**
HÜFTPFANNENIMPLANTAT FÜR HÜFTPROTHESEN
ACETABULAR IMPLANT FOR HIP PROSTHESIS

(30) Priorité: 04.03.2020 FR 2002164
(43) Date de publication de la demande: 11.01.2023
(73) Titulaire: Giles, 69300 Caluire-Et-Cuire (FR)
(72) Inventeur: NOYER, Daniel, 38200 Luzinay (FR); BAUCHU, Philippe, 69002 Lyon (FR); CYPRES, Alain, 42190 Saint-Nizier-sous-Charlieu (FR); FIQUET, Arnaud, 69300 Caluire-Et-Cuire (FR); ROY, Christophe, 26300 Chatuzange-Le-Goubet (FR); BONNARD, Olivier, 69330 Meyzieu (FR); GIRARDIN, Philippe, 42600 Lezigneux (FR); SEUTIN, Bertrand, 26160 La Batie-Rolland (FR); VAZ, Gualter, 69002 Lyon (FR); ROBERTS, Philip John, Nantwich, Cheshire CW58PJ (GB)
(74) Mandataire: Vuillermoz, Bruno
(86) Numéro de dépôt international: PCT/FR2020/052473
(87) Numéro de publication internationale: WO 2021/176148

(56) Documents cités:
- EP-A1- 0 461 019
- FR-A1- 2 795 302

## Description

### DOMAINE DE L'INVENTION

L'invention concerne un implant acétabulaire pour prothèse de hanche, du type à double mobilité.

### ETAT ANTERIEUR DE LA TECHNIQUE

Les prothèses totales de hanche, dites à double mobilité, sont d'un usage aujourd'hui largement répandu. Pour l'essentiel, ce principe de la double mobilité vise la rotation d'une tête fémorale prothétique ou bille prothétique, fixée sur une tige prothétique insérée dans le fémur, au sein d'un insert mobile, ce dernier étant à son tour également mobile en rotation au sein d'une cupule ou implant acétabulaire ancré(e), et de manière générale fixée dans la cavité acétabulaire du bassin du patient à prothéser.

Il est ainsi défini deux articulations, respectivement une petite articulation (tête fémorale prothétique / insert mobile) et une grande articulation (insert mobile / cupule), d'où le terme générique de double mobilité. Traditionnellement, la petite articulation est mise en oeuvre dans les mouvements de faible amplitude, alors que la grande articulation se met en action lorsque le col fémoral de la tige prothétique vient au contact du rétreint, dont est traditionnellement muni l'insert mobile, lors des mouvements de grande amplitude.

Cette double mobilité a permis de diminuer de manière importante les luxations de l'articulation ainsi prothésée. Une telle prothèse a par exemple été décrite dans le document FR 2 710 836 ou encore FR 2 948 013. Le préambule de la revendication 1 est défini par le document EP 0461019 A1.

L'utilisation de telles prothèses dites à double mobilité a significativement réduit le nombre de luxations des articulations ainsi prothésées et, corollairement, augmenté leur durée de vie. Il n'en demeure pas moins vrai qu'un certain nombre de problèmes subsistent, en raison de la présence d'une double surface articulaire, respectivement de la petite articulation et de la grande articulation, et générant, en raison des surfaces de contact respectives et nonobstant les progrès réalisés en termes de matériaux constitutifs, des phénomènes de luxations ou encore de blocage, conduisant à l'échec prothétique.

Par ailleurs, la fixation de la cupule d'ancrage au sein de la cavité acétabulaire constitue systématiquement un point délicat pour la pérennité de la prothèse alors implantée. Ainsi, afin d'optimiser cette fixation de la cupule d'ancrage, on a développé le principe de cupules métalliques d'ancrage, éventuellement à expansion, munies de moyens de fixation, tels que par exemple des pattes de fixation, avec un grand nombre de variantes. La littérature en la matière est abondante, mais une tendance qui tend à se dégager concerne la mise en oeuvre d'une cupule d'ancrage systématiquement métallique, se traduisant de manière générale par un surenchérissement des coûts de fabrication, voire d'implantation. Ces coûts se heurtent donc à la tendance actuelle des organismes étatiques ou des officines complémentaires de prise en charge de ces frais.

En outre, les cupules d'ancrage actuellement mises en oeuvre dans le cadre des prothèses à double mobilité présentent une convexité métallique et sont destinées à être implantées majoritairement au sein de la cavité acétabulaire sans ciment, et déclinées pour certaines en version métallique à sceller avec du ciment. Cependant, les cupules réalisées en polyéthylène, matériau biocompatible, et destinées à être scellées avec du ciment au sein de ladite cavité acétabulaire demeurent à ce jour une référence aux yeux de nombreux chirurgiens orthopédistes de par le monde, qui disposent de fait du recul suffisant en termes de modalités de fixation dans l'os par l'intermédiaire du ciment pour apprécier l'opportunité de leur mise en oeuvre.

On connait également une prothèse à double mobilité, mettant en oeuvre une cupule réalisée en polyéthylène, fixée par ciment au sein de la cavité acétabulaire, et dont l'insert mobile est également réalisé en polyéthylène. Cependant, cet insert mobile est blindé au moyen d'un revêtement métallique au niveau de sa surface externe, c'est-à-dire au niveau de sa zone de coopération par liaison rotule avec la cavité interne de la cupule d'ancrage. Ce blindage a pour vocation de favoriser le fonctionnement de la grande articulation, car on évite ainsi la coopération de deux surfaces réalisées en polyéthylène entre elles. En raison de ce blindage, le poids de l'insert mobile s'en trouve augmenté, tendant à entrainer sa bascule permanente contre la tige avec laquelle il coopère par le biais de la petite articulation avec la bille prothétique ou tête fémorale prothétique fixée sur ladite tige, entrainant une usure prématurée du rétreint de l'insert mobile.

L'objet de l'invention est de proposer une telle prothèse de hanche, plus particulièrement un implant acétabulaire double mobilité selon la définition du genre, pour prothèse de hanche, d'une part d'un coût de revient limité, et d'autre part permettant de s'affranchir des problèmes subsistants au niveau des deux articulations constitutives de la double mobilité.

### EXPOSE SOMMAIRE DE L'INVENTION

Ainsi, l'invention vise un implant acétabulaire pour prothèse de hanche comprenant :
▪ une cupule d'ancrage à cimenter dans la cavité acétabulaire du bassin du patient à prothéser, définissant une cavité interne de forme sensiblement hémisphérique ;
▪ un insert mobile au sein de ladite cavité interne, et dont la surface externe lisse est destinée à coopérer par liaison rotule avec ladite cavité interne de la cupule d'ancrage, et définissant à son tour une cavité interne lisse également de forme sensiblement hémisphérique ;
▪ une tête ou bille, solidarisée sur une tige fémorale, de forme sphérique et destinée à être reçue dans la cavité interne de l'insert mobile, et à constituer une seconde liaison rotule.

L'invention est définie dans la revendication 1.

Selon l'invention :
▪ la cupule d'ancrage est réalisée en polyéthylène et présente, sur sa paroi externe, des excroissances ou saillies, et de manière générale des moyens aptes à favoriser son scellement par ciment au sein de la cavité acétabulaire ;
▪ la cavité interne de ladite cupule d'ancrage est revêtue d'un blindage en matériau à dureté élevée, par exemple en un alliage de type chrome-cobalt ou zirconium oxydé, acier inoxydable, céramique, PEEK (polyétherethercétone), etc..., et de manière générale tout matériau biocompatible de dureté élevée, ledit blindage définissant la cavité interne lisse de forme sensiblement hémisphérique destinée à recevoir l'insert mobile ;
▪ l'insert mobile est réalisé en polyéthylène haute densité.

En d'autres termes, l'invention consiste tout d'abord à permettre de sceller la cupule d'ancrage avec du ciment dans la cavité acétabulaire, comme déjà dit, réalisée en polyéthylène haute densité, opération de scellement facilitée par les excroissances ou saillies dont est pourvue sa surface externe. Elle consiste ensuite à blinder ladite cupule d'ancrage par un matériau biocompatible à dureté élevée, et typiquement en métal, en acier inoxydable, en PEEK, en céramique ou en alliage de zirconium oxydé du type commercialisé sous la marque Oxynium, ce, dans le but d'optimiser la mobilité de l'insert mobile au sein de la grande articulation, en limitant notamment la genèse de déchets et autres défauts susceptibles de bloquer cette articulation.

### BREVE DESCRIPTION DES FIGURES

La manière dont l'invention peut être réalisée et les avantages qui en découlent ressortiront mieux de l'exemple de réalisation qui suit, donné à titre indicatif et non limitatif, à l'appui des figures annexées.
La figure 1 est une représentation schématique en section sagittale de l'implant acétabulaire conforme à l'invention.
La figure 2 est une vue en éclaté de l'implant de la figure 1.
La figure 3 est une vue schématique en perspective d'un mode particulier de réalisation de la cupule d'ancrage conforme à l'invention.
La figure 4 est vue schématique en section sagittale de l'implant acétabulaire conforme à un autre mode de réalisation de l'invention.

### DESCRIPTION DETAILLEE DE L'INVENTION

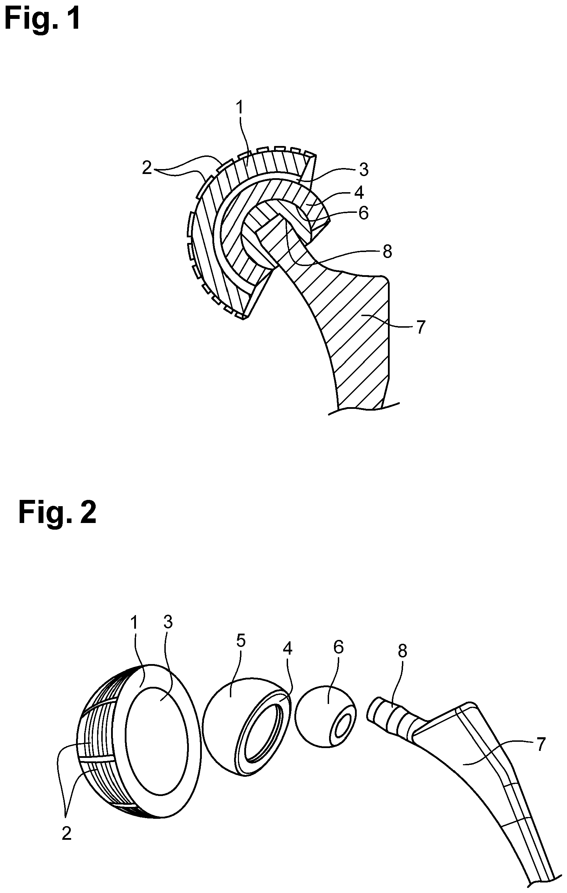

On a donc représenté, en relation avec les figures 1 et 2, un implant acétabulaire conforme à un premier mode de réalisation de l'invention.

Fondamentalement, il se compose tout d'abord, d'une cupule d'ancrage (1), réalisée en polyéthylène haute densité. Cette cupule d'ancrage est pourvue à sa surface externe de moyens d'ancrage (2), typiquement constitués de saillies, sillons, et de manière générale de tout dispositif apte à favoriser le scellement avec du ciment, de ladite cupule d'ancrage au sein de la cavité acétabulaire de l'articulation considérée.

Selon l'invention, cette cupule d'ancrage présente un blindage articulaire (3) au niveau de la cavité interne de forme hémisphérique qu'elle définit. Typiquement, ce blindage peut être tout d'abord usiné, puis encastré ensuite au sein de la cupule en polyéthylène. Alternativement, il peut être embouti, puis surmoulé au moyen de polyéthylène constitutif de ladite cupule. Ce blindage peut également être forgé, puis usiné, avant là encore d'être surmoulé par le polyéthylène.

Typiquement, ce blindage est réalisé en un matériau à dureté élevée, tel que par exemple en acier, notamment acier inoxydable, ou encore en alliage chrome-cobalt, ou encore en céramique ou encore en alliage à base de zirconium oxydé, tel que commercialisé sous la marque Oxinium^{®}. Quelle que soit la nature de ce blindage, celui-ci est bien évidemment de nature biocompatible.

De ce fait, le blindage (3) et la cupule d'ancrage (1) en polyéthylène constituent une seule et même pièce destinée à être implantée au niveau de la cavité acétabulaire au préalable préparée par fraisage. La fixation est assurée par scellement au ciment chirurgical.

Comme déjà évoqué, ce blindage (3) participe à la grande articulation, c'est-à-dire entre la cupule d'ancrage et l'insert mobile. Le couple de frottement entre l'insert mobile, en polyéthylène, et la cupule d'ancrage, scellée avec du ciment au sein de la cavité acétabulaire, intervient donc entre le matériau dur constitutif du blindage (3) de la cavité interne de ladite cupule d'ancrage (1) et le polyéthylène constitutif de l'insert mobile (4). Il est connu de l'art antérieur que le couple de frottement polyéthylène / polyéthylène est voué à un échec rapide en raison de mauvaises conditions tribologiques. L'apport du blindage en matériau dur de l'invention, vise tout d'abord à proposer le même coefficient de friction au niveau de la grande articulation qu'à celui de la petite articulation (bille prothétique ou tête fémorale prothétique rapportée sur la tige fémorale, en métal ou céramique ou Oxinium / cavité interne de l'insert mobile en polyéthylène).

En outre, la mise en oeuvre d'un blindage réalisé en un matériau à dureté élevée, apporte une meilleure répartition des contraintes dans les zones de l'implant acétabulaire réalisé en polyéthylène, permettant de s'affranchir des problèmes de fluage ou équivalent. Ledit blindage agit ainsi comme une barrière tendant à s'opposer à la-déformation de l'insert mobile, ce dernier venant épouser la partie concave dudit blindage, et la cupule en polyéthylène à cimenter, solidaire de ce blindage, venant épouser la partie convexe de ce dernier, en particulier lorsque que l'ensemble est sous charge au moment par exemple de l'attaque du pied sur le sol réalisant la mise en charge monopodale complète de l'implant.

Par ailleurs, la mise en oeuvre d'un tel blindage confère un pouvoir d'amortissement à l'articulation ainsi prothésée. En effet, le blindage, d'épaisseur relativement faible, et typiquement de l'ordre de 0,8 à 3 millimètres, sans que cette précision ne revête une quelconque limitation de l'invention, mais dépendant des caractéristiques du matériau utilisé, se retrouve une fois l'implant mis en place, entre deux épaisseurs de polyéthylène. Ainsi, par exemple lors de l'attaque du pied concerné, la pression engendrée tend tout d'abord à déformer l'insert mobile qui vient de fait prendre appui complet contre le blindage. Ce dernier restitue à son tour cette charge à la partie de la cupule d'ancrage en polyéthylène. Il en résulte qu'aux effets inhérents à la mise en oeuvre du principe de la double mobilité, qui préserve des effets de cisaillements de l'ancrage acétabulaire, en raison du découplage des frictions mises enjeu lors des mouvements, s'ajoute la préservation de l'ancrage cimenté en raison de cet amortissement, contribuant ainsi à un bienfait notable pour le patient.

La cavité interne définie par le blindage articulaire concave (3), est destinée à recevoir un insert mobile (4) réalisé en polyéthylène haute densité, de forme externe (5) convexe et hémisphérique. Le diamètre externe de l'insert mobile (4) correspond, au jeu près, au diamètre interne du blindage concave (3). Il se définit ainsi une première surface articulaire entre la cupule d'ancrage (1) et l'insert mobile (4).

Ce dernier (4) définit également une cavité interne, là encore de forme hémisphérique, destinée à coopérer par liaison rotule avec une tête ou bille fémorale (6) réalisée en acier, voire en céramique ou en zirconium oxydé), là encore de qualité biocompatible. Le diamètre externe de la bille prothétique (6) correspond là encore, au jeu près, au diamètre interne de l'insert mobile (4).

Cette tête fémorale prothétique ou bille prothétique est destinée, de manière connue, à venir se fixer à l'extrémité d'une tige fémorale (7), par exemple munie d'un cône morse (8), ce, de manière connue. Il pourrait cependant être envisagé la mise en oeuvre d'une tige fémorale monobloc avec une tête de taille adaptée.

En raison de la conception d'un tel implant particulier, on conçoit tout d'abord l'optimisation du fonctionnement de l'articulation, et corollairement un plus grand confort pour le patient.

Par ailleurs, en raison de la double mobilité, et des deux couches de polyéthylène, l'ancrage cimenté est moins sollicité, notamment en cisaillement et compression, permettant de pérenniser l'ancrage osseux, et donc corollairement, la prothèse ainsi implantée.

En outre, le polyéthylène, constitutif de la cupule d'ancrage ou de l'insert mobile n'est plus soumis au frottement avec un élément également en polyéthylène en raison du blindage, et donc corollairement à l'usure subséquence, il n'y a plus libération de particules d'usure au sein de l'articulation et au sein de l'os péri-articulaire

De plus, un tel implant est plus facilement implantable au niveau d'une cavité acétabulaire susceptible de présenter un défaut osseux important, puisqu'aussi bien, ledit défaut osseux peut être comblé avec le ciment de fixation de la cupule. Dans la même idée, cet implant peut également être cimenté au sein d'un anneau de reconstruction acétabulaire (type croix de Kerboull, anneau de Ganz ou Muller ou Bursch-Schneider ou tout autre système métallique de reconstruction acétabulaire), particulièrement dans le cas de situation de précarité du stock osseux acétabulaire.

Enfin, le prix de réalisation de l'implant de l'invention est réduit en raison de la nature des matériaux constitutifs et de leurs modes de mise en oeuvre.

Il convient en outre de préciser qu'en combinant les enseignements de la présente invention avec ceux décrits dans la demande de brevet français FR 1902133 non publiée à la date de dépôt de la présente demande, on optimise la fiabilité de l'implant en question, en raison d'une part, des résultats inhérents à la présente invention, et d'autre part, en réduisant le différentiel de couple entre la petite articulation et la grande articulation consécutif aux caractéristiques décrites dans cette autre demande.

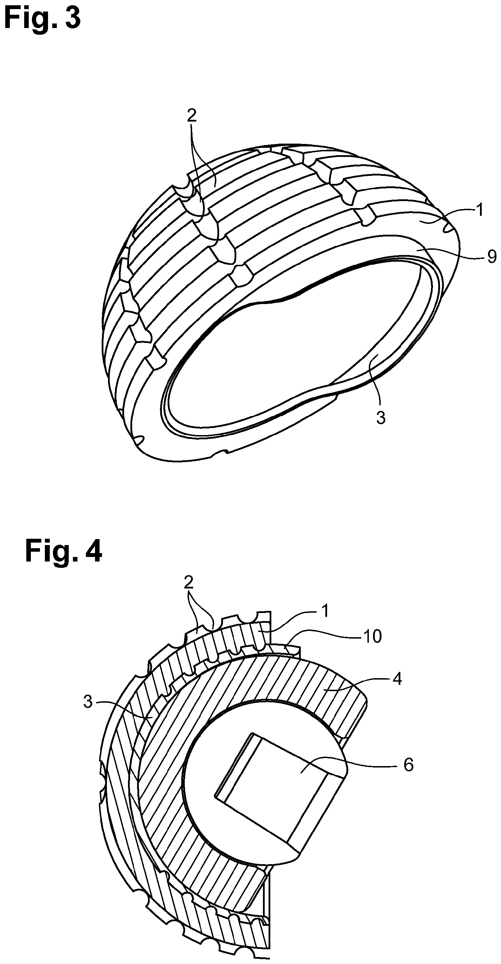

Dans l'exemple de réalisation illustré au sein des figures 1 et 2, la cupule d'ancrage acétabulaire est exempte de tout rebord anti-luxation, comme d'ordinaire sur les prothèses dites à double mobilité standard. On a représenté au sein de la figure 3 un autre mode de réalisation de la présente invention, en incluant un tel rebord anti-luxation (9). Ce rebord (9) peut être de diverses conceptions possibles, et notamment résulter soit du blindage (3), soit de la cupule (1) proprement dite, soit des deux ensembles, voire même être rapporté. Ce faisant, dans les trois premiers cas, il est monobloc et est réalisé dans le matériau constitutif de l'élément (cupule ou blindage) duquel il émane, c'est-à-dire soit en polyéthylène, soit en matériau biocompatible à dureté élevée du type de ceux précédemment évoqué.

Par ailleurs, dans certaines pathologies plus rares, comme celles présentant des déficiences musculaires importantes, il peut être opportun pour le chirurgien de pouvoir rendre rétentif la grande articulation, c'est-à-dire, d'empêcher la dé-coaptation de l'insert mobile (4) hors de la cavité interne hémisphérique définie par le blindage (3). Cette configuration particulière permet de disposer d'une liaison sensiblement rotoïde avec éventuellement un peu de translation potentielle pour autoriser le « pumping articulaire », c'est-à-dire, grâce au jeu existant au sein de chacune des petite et grande articulations, le fonctionnement normal de l'articulation ainsi prothésée. Cela permet au patient d'avoir une articulation prothétique à grand débattement, inhérent à la double mobilité avec tous les autres avantages énumérés ci-dessus, tout en limitant le risque de luxation susceptible de résulter de la déficience musculaire qu'il présente.

On a de fait représenté au sein de la figure 4 une forme de réalisation de l'implant acétabulaire de l'invention à double mobilité dite rétentive. Dans celui-ci, la cupule d'ancrage (1) comprend un moyen de rétention de l'insert mobile (4), apte à laisser libres les mouvements de circumduction, mais à s'opposer aux mouvements de translation dudit insert mobile. Typiquement, ce moyen de rétention résulte du blindage (3), qui s'étend au-delà de l'équateur de la demi-sphère qu'il définit, constituant ainsi un rebord périphérique (10), apte à s'opposer au retrait de l'insert mobile (4). Ce dernier, préalablement équipé de sa tête fémorale prothétique, est introduit en force au sein de la cupule d'ancrage munie de son blindage.

## Revendications

1. Implant acétabulaire pour prothèse de hanche comprenant :
▪ une cupule d'ancrage (1) dans la cavité acétabulaire du bassin du patient à prothéser, définissant une cavité interne de forme sensiblement hémisphérique ;
▪ un insert (4) mobile au sein de ladite cavité interne, et dont la surface externe lisse est destinée à coopérer par liaison rotule avec ladite cavité interne de la cupule d'ancrage (1), et définissant à son tour une cavité interne lisse également de forme sensiblement hémisphérique ;
▪ une tête fémorale prothétique ou bille prothétique (6), solidarisée sur une tige fémorale (7), de forme sphérique et destinée à être reçue dans la cavité interne de l'insert mobile (4), et à constituer une seconde liaison rotule;
▪ la cupule d'ancrage (1) étant réalisée en polyéthylène et présentant, sur sa paroi externe, des excroissances ou saillies (2), et de manière générale des moyens aptes à favoriser sa fixation par ciment au sein de la cavité acétabulaire du bassin du patient à prothéser, *caractérisé*
▪ en ce que la cavité interne de ladite cupule d'ancrage (1) est revêtue d'un blindage (3) réalisé en un matériau biocompatible à dureté élevée, ledit blindage définissant la cavité interne lisse de forme sensiblement hémisphérique destinée à recevoir l'insert mobile (4) ;
▪ et en ce que l'insert mobile (4) est réalisé en polyéthylène haute densité.

2. Implant acétabulaire pour prothèse de hanche selon la revendication 1, dans lequel le matériau constitutif du blindage (3) est réalisé en métal, en acier inoxydable, en alliage chrome-cobalt, en céramique, en PEEK ou en alliage de zirconium oxydé.

3. Implant acétabulaire pour prothèse de hanche selon l'une des revendications 1 et 2, dans lequel la cupule d'ancrage (1) est munie de moyens supplémentaires anti-luxation (9), monoblocs ou rapportés sur ladite cupule d'ancrage.

4. Implant acétabulaire pour prothèse de hanche selon la revendication 3, dans lequel les moyens supplémentaires anti-luxation (9) sont réalisés en polyéthylène ou en un matériau biocompatible à dureté élevée.

5. Implant acétabulaire pour prothèse de hanche selon l'une des revendications 1 à 4, dans lequel la cupule d'ancrage (1) comprend un moyen de rétention (10) de l'insert mobile (4), apte à laisser libres les mouvements de circumduction, mais à s'opposer aux mouvements de translation dudit insert mobile.

6. Implant acétabulaire pour prothèse de hanche selon la revendication 5, dans lequel le moyen de rétention de l'insert mobile (4) est constitué par une prolongation du blindage (3) au-delà de la demi-sphère qu'il définit.

## Patentansprüche

1. Hüftgelenkpfannenimplantat für eine Hüftprothese, umfassend:
▪ eine Verankerungspfanne (1) in der zu prothetisierenden Hüftgelenkpfanne des Beckens des Patienten, die einen inneren Hohlraum von im Wesentlichen halbkugelförmiger Form definiert;
▪ einen Einsatz (4), der innerhalb des inneren Hohlraums beweglich ist und dessen glatte Außenfläche dazu bestimmt ist, über eine Kugelgelenkverbindung mit dem inneren Hohlraum der Verankerungspfanne (1) zusammenzuwirken, und der wiederum einen glatten inneren Hohlraum definiert, der ebenfalls eine im Wesentlichen halbkugelförmige Form aufweist;
▪ einen prothetischen Femurkopf oder eine prothetische Kugel (6), die an einem Femurschaft (7) befestigt ist, kugelförmig ist und dazu bestimmt ist, im inneren Hohlraum des beweglichen Einsatzes (4) aufgenommen zu werden und eine zweite Gelenkverbindung zu bilden,
▪ wobei die Verankerungspfanne (1) aus Polyethylen hergestellt ist und an ihrer Außenwand Vorsprünge oder Erhebungen (2) sowie im Allgemeinen Mittel aufweist, die ihre Fixierung mit Zement in der zu prothetisierenden Hüftgelenkpfanne des Beckens des Patienten begünstigen; **dadurch gekennzeichnet**,
▪ dass der innere Hohlraum der Verankerungspfanne (1) mit einer Abschirmstruktur (3) ausgekleidet ist, die aus einem biokompatiblen Material hoher Härte hergestellt ist, wobei die Abschirmstruktur den glatten inneren Hohlraum von im Wesentlichen halbkugelförmiger Form definiert, der dazu bestimmt ist, den beweglichen Einsatz (4) aufzunehmen;
▪ und dass der bewegliche Einsatz (4) aus hochdichtem Polyethylen hergestellt ist.

2. Hüftgelenkpfannenimplantat für eine Hüftprothese nach Anspruch 1, wobei das die Abschirmstruktur (3) bildende Material aus Metall, aus Edelstahl, aus einer Chrom-Kobalt-Legierung, aus Keramik, aus PEEK oder aus einer oxidierten Zirkoniumlegierung hergestellt ist.

3. Hüftgelenkpfannenimplantat für eine Hüftprothese nach einem der Ansprüche 1 und 2, wobei die Verankerungspfanne (1) mit zusätzlichen Antiluxationsmitteln (9) versehen ist, die mit der Verankerungspfanne einstückig oder an dieser angebracht sind.

4. Hüftgelenkpfannenimplantat für eine Hüftprothese nach Anspruch 3, wobei die zusätzlichen Antiluxationsmittel (9) aus Polyethylen oder einem biokompatiblen Material hoher Härte hergestellt sind.

5. Hüftgelenkpfannenimplantat für eine Hüftprothese nach einem der Ansprüche 1 bis 4, wobei die Verankerungspfanne (1) ein Rückhaltemittel (10) für den beweglichen Einsatz (4) umfasst, das dazu ausgelegt ist, die Zirkumduktionsbewegungen frei zu lassen, aber den Translationsbewegungen des beweglichen Einsatzes entgegenzuwirken.

6. Hüftgelenkpfannenimplantat für eine Hüftprothese nach Anspruch 5, wobei das Rückhaltemittel für den beweglichen Einsatz (4) durch eine Verlängerung der Abschirmstruktur (3) über die von ihr definierten Halbkugel hinaus ausgebildet ist.

## Claims

1. An acetabular implant for hip prosthesis comprising:
▪ an anchoring cup (1) in the acetabular cavity of the patient's pelvis to be prosthesised, defining an inner cavity which is also substantially hemispherical in shape;
▪ an insert (4) which is movable within said inner cavity, and whose smooth outer surface is intended to cooperate by ball joint connection with said inner cavity of the anchoring cup (1), and in turn defining a smooth inner cavity which is also substantially hemispherical in shape;
▪ a prosthetic femoral head or prosthetic ball (6), secured to a femoral stem (7), of spherical shape and intended to be received in the inner cavity of the movable insert (4), and to constitute a second ball joint;
▪ the anchoring cup (1) being made of polyethylene and having, on the outer wall thereof, protrusions or projections (2), and generally means capable of promoting the fastening thereof by cement within the acetabular cavity of the patient's pelvis to be prosthesised, characterised
▪ in that the inner cavity of said anchor cup (1) is coated with a shield (3) made of a biocompatible material with high hardness, said shield defining the smooth inner cavity which is also substantially hemispherical in shape intended to receive the movable insert (4);
▪ and in that the movable insert (4) is made of high-density polyethylene.

2. The acetabular implant for hip prosthesis according to claim 1, wherein the material constituting the shield (3) is made of metal, stainless steel, chrome-cobalt alloy, ceramic, PEEK or oxidised zirconium alloy.

3. The acetabular implant for hip prosthesis according to one of claims 1 and 2, wherein the anchoring cup (1) is provided with additional anti-dislocation means (9), in one piece or directly mounted on said anchoring cup.

4. The acetabular implant for hip prosthesis according to claim 3, wherein the additional anti-dislocation means (9) are made of polyethylene or of a high hardness biocompatible material.

5. The acetabular implant for hip prosthesis according to one of claims 1 to 4, wherein the anchoring cup (1) comprises means (10) for retaining the movable insert (4), capable of leaving free the movements of circumduction, but of opposing the translational movements of said movable insert.

6. The acetabular implant for hip prosthesis according to claims 5, wherein the means for retaining the movable insert (4) is constituted by an extension of the shield (3) beyond the half-sphere that he defines.
